(19) Europäisches Patentamt

European Patent Office

Office européen des brevets

(11) Veröffentlichungsnummer: **0 463 615 A1**

(12) **EUROPÄISCHE PATENTANMELDUNG**

(21) Anmeldenummer: **91110553.4**

(22) Anmeldetag: **26.06.91**

(51) Int. Cl.5: **C12P 21/02**, A61K 37/38, A61K 37/02, C07K 7/06

(30) Priorität: **26.06.90 DD 342077**

(43) Veröffentlichungstag der Anmeldung:
**02.01.92 Patentblatt 92/01**

(84) Benannte Vertragsstaaten:
**AT BE CH DE ES FR GB GR IT LI NL SE**

�]1 Anmelder: **BERLIN-CHEMIE AG**
**Glienicker Weg 125-127**
**O-1199 Berlin(DE)**

(72) Erfinder: **Hänsicke, Andre, Dr. rer. nat.**
**Thelenstrasse 12**
**O-1199 Berlin(DE)**
Erfinder: **Bienert, Michael, Dr. sc. nat.**
**Parkstrasse 14**
**O-1114 Berlin(DE)**
Erfinder: **Krause, Eberhard, Dr. rer. nat.**
**Assmannstrasse 49**
**O-1162 Berlin(DE)**
Erfinder: **Jakubke, Hans-Dieter, Prof. Dr. sc. nat.**
**Johannes-R.-Becher-Strasse 4**
**O-7030 Leipzig(DE)**
Erfinder: **Schellenberger, Volker, Dr. rer. nat.**
**Theresienstrasse 39**
**O-7021 Leipzig(DE)**
Erfinder: **Schellenberger, Ute, Dipl.-Chem.**
**Theresienstrasse 39**
**O-7021 Leipzig(DE)**

(74) Vertreter: **Hansen, Bernd, Dr. Dipl.-Chem. et al**
**Hoffmann, Eitle & Partner Patent- und**
**Rechtsanwälte Arabellastrasse 4 Postfach**
**81 04 20**
**W-8000 München 81(DE)**

(54) **Verfahren zur Herstellung von GnRH-Dekapeptiden.**

㊼ Die Erfindung betrifft ein Verfahren zur Herstellung von GnRH-Dekapeptiden. Die Aufgabe wird dadurch gelöst, daß die in an sich bekannter Weise hergestellten Teilfragmente

Pyr-His-Trp-OR und
H-Ser-Tyr-Xaa-Leu-Arg-Pro-Gly-NH$_2$,

wobei R einen aliphatischen oder aromatischen Rest und Xaa eine D-Aminosäure bedeuten, erfindungsgemäß mittels Chymotrypsin gekuppelt werden. Das Verfahren gewährleistet eine racemisierungsfreie Herstellung der GnRH-Dekapeptide in hoher Ausbeute und Reinheit. Die Anwendungsgebiete der Erfindung liegen in der Herstellung von Arzneimitteln, die zur Behandlung von Fertilitätsstörungen, Prostatakarzinom oder zur Ovulationssynchronisation eingesetzt werden.

Die Erfindung betrifft ein neues Verfahren zur Herstellung von Dekapeptiden, speziell des GnRH

Pyr-His-Trp-Ser-Tyr-Xaa-Leu-Arg-Pro-Gly-NH$_2$    I,

wobei Xaa = DPhe oder eine andere D-Aminosäure bedeutet. Die Peptide eignen sich zur Herstellung von Arzneimitteln, die zur Behandlung von Fertilitätsstörungen, Prostatakarzinom oder zur Ovulationssynchronisation eingesetzt werden. Die Anwendungsgebiete der Erfindung liegen in der Humanmedizin sowie in der Veterinärmedizin.

Die Synthese von GnRH sowie von GnRH-Analoga ist mehrfach beschrieben worden. Die Synthesen erfolgen sowohl in homogener Phase als auch durch Aufbau an polymeren Trägern. Der Aufbau der Peptide in Lösung erfolgt über Teilsequenzen, z.B. Pyr-His-NHNH$_2$, Pyr-His-Trp-OH, Pyr-His-Trp-NHNH$_2$, H-Ser($^t$Bu)-Tyr-Gly-Leu-OH, H-Trp-Ser($^t$Bu)-Tyr-Gly-Leu-OH, H-Arg-Pro-Gly-NH$_2$, H-Arg(NO$_2$)-Pro-Gly-NH$_2$ oder H-Arg(TOS)-Pro-Gly-NH$_2$ (DE 23 07 010, DE 24 24 287, Tetrahedron Letters 41, 4071 (1973); Coll. Czech. Chem. Commun. 42 2018 (1976); J. Med. Chem. 15, 222 (1972); Chem. Pharm. Bull. 23, 229 (1975); J. Chem. Soc. Perkin Trans. I, 1979, 389; Helv. Chim. Acta 57, 231 (1974); J. Med. Chem. 17, 1060 (1974); J. Med. Chem. 15, 623 (1972); J. Med. Chem. 18, 613 (1975); US 3 953 416; Chem. Pharm. Bull. 27, 1907 (1979); Peptides 1972 (Ed. H. Hanson, H.D. Jakubke), North Holland Publ. Amsterdam 1973, S. 93). Die Kupplungen der Fragmente werden teils über Azidsynthesen und teils über DCC-Kondensationen realisiert, wobei von einigen Autoren racemisierungsvermindernde Reagenzien wie HOBt, HOOBt, HONB, HONSu zugesetzt werden. Es ist auch bekannt, die Kupplung von Teilsequenzen wie Pyr-His-Trp-OH mittels DCC ohne Additiva durchzuführen (Helv. Chim. Acta. 57, 231 (1974), US 3 953 416). Über das Ausmaß einer Racemisierung an der C-term. aktivierten Aminosäuren wurden allerdings keine Angaben gemacht. Bei Untersuchungen zur Kupplung von Pyr-His-Trp-OH wurden bei allen DCC/Additiva-Varianten 5 bis 15 % D-Trp-Peptid im Endprodukt gefunden. Zur Vermeidung des Racemisierungsrisikos wird von anderen Autoren deshalb die Azidsynthese trotz der dabei oft unbefriedigenden Ausbeuten oder ein schrittweiser Aufbau des Fragmentes 1 bis 6 gewählt. Beim schrittweisen Aufbau der GnRH-Peptide durch Festphasensynthese ist eine Racemisierung ebenfalls weitgehend ausgeschlossen. Lediglich für Histidin-Derivate ist in Abhängigkeit vom verwendeten Seitenkettenschutz Racemisierung zu erwarten.

Nachteilig bei den durchgeführten Festphasensynthesen, vor allem bei Synthesen im Produktionsmaßstab, sind Nebenreaktionen, die vor allem durch die hohe Säurelabilität und Alkylierbarkeit des Tryptophan-Restes, durch die hohe Racemisierungstendenz von einigen Histidinderivaten, durch N$^{1m}$-N$^α$-Wanderung von N$^{1m}$-Schutzgruppen sowie durch unvollständige Kupplungen im N-Terminus verursacht werden. Dies führte zu der Strategie, ein nach klassischen Methoden in Lösung hergestelltes Tripeptid an ein durch Festphasensynthese aufgebautes Heptapeptid vor der Abspaltung vom Harz (DD 135 078) oder nach Abspaltung [Ann. d' Endocrinol. (Paris) 37, 215 (1976] zu kondensieren. Nachteilig hierbei ist allerdings wiederum die am C-terminalen Trp zu beobachtende Racemisierung.

Weiterhin ist bekannt, daß Peptidfragmente durch enzymatische Katalyse racemisierungsfrei verknüpft werden können [Angew. Chem. Int. Ed. 24, 85 (1985)].

So wird eine GnRH-Synthese beschrieben, die nahezu ausschließlich auf enzymatischen Kupplungsschritten beruht (Peptides 1986; Ed. D. Theodoropoulos, W. de Gruyter, Berlin 1987, S. 183): Der N-term. Tripeptid-ester Pyr-His-Trp-OMe wird mittels Chymotrypsin und Serinethylester zum Tetrapeptid Pyr-His-Trp-Ser-OEt verlängert. In weiteren Kupplungsschritten kommen Carboxypeptidase Y, Carboxypeptidase YM, Post-Prolin-spezifische Endoprotease und Chlostripain, die allerdings im Vergleich zu Chymotrypsin sehr teuer sind, zur Anwendung. Nachteilig bei der Verwendung dieser ausschließlich enzymatischen Kupplungsstrategie ist auch der hohe Optimierungsaufwand vor allem zur Vermeidung uner-wünschter Peptidspaltungen, da alle fünf verwendeten Enzyme unterschiedliche Reaktionsbedingungen für Kupplung und Spaltung erfordern.

Zweck der Erfindung ist es, ein einfaches und ökonomisches Verfahren zur industriellen Herstellung von GnRH-Peptiden, Formel I, zu entwickeln.

Der Erfindung liegt die Aufgaben zugrunde, ein einfaches Verfahren zur Herstellung von GnRH-Peptiden, Formel I,

Pyr-His-Trp-Ser-Tyr-Xaa-Leu-Arg-Pro-Gly-NH$_2$    I,

zu entwickeln, nach dem die Peptide auch im industriellen Maßstab in hoher Ausbeute und Reinheit ohne Racemisierung herstellbar sind. Die Aufgabe wird dadurch gelöst, daß die in an sich bekannter Weise hergestellten Teilfragmente der Formeln II und III

Pyr-His-Trp-Or    II,

H-Ser-Tyr-Xaa-Leu-Arg-Pro-Gly-NH$_2$,    III,

wobei R einen aliphatischen oder aromatischen Rest, vorzugsweise -CH$_3$, –C$_2$H$_5$ und Xaa eine D-Aminosäure, vorzugsweise D-Phe, D-Ala, D-Trp, D-Leu, D-ß-Nal, D-Ser($^t$Bu) bedeuten, erfindungsgemäß mittels Chymotrypsin gekuppelt werden.

Die enzymkatalysierte Segmentsynthese erfolgt vorteilhaft in einem Gemisch aus Dimethylformamid und Wasser, wobei die Reaktion in einem Lösungsmittelgemisch aus 30 bis 60 % DMF bei einer Temperatur von -10 °C bis 35 °C und bei einem pH-Wert von 7,8 bis 8,9 durchgeführt wird. Die Reaktionsgeschwindigkeit wird durch den gewählten Temperaturbereich und die eingesetzte Enzymmenge bestimmt, wobei die Reaktionszeit bis zu 2 Tagen, vorzugsweise 4 bis 8 Stunden beträgt.

Das erfindungsgemäße Verfahren gewährleistet eine racemisierungsfreie Herstellung der GnRH-Dekapeptide in hoher Ausbeute und Reinheit. Bei der enzymatischen Peptidsynthese, insbesondere bei der Kupplung von Peptidfragmenten, zu erwartende Schwierigkeiten durch auftretende Nebenspaltungen finden nicht statt. Aufgrund der bekannten Spezifität des Chymotrypsins für Reaktionen an Carboxylgruppen aromatischer Aminosäuren ist es überraschend, daß bei der erfindungsgemäßen Kupplung die Tyr-Xaa-Peptidbindung nicht hydrolytisch durch Chymotrypsin gespalten wird.

Das Verfahren weist eine Reihe weiterer Vorteile auf:
Dem Aufbau der Dekapeptide liegen Fragmente zugrunde, die durch klassische Synthesemethoden in Lösung (Fragment II: z.B. DE 23 07 010) sowie durch Festphasensynthese (Fragment III, z.B. DD 135 078) gut herstellbar sind. So kann das Heptapeptid III unter Verwendung von $N^\alpha$-Boc geschützten Aminosäuren, Arg($NO_2$), Ser(Bzl) Seitenkettenschutz am Benzhydrylamin-Harz mittels DCC/HOBt problemlos im 50-bis-1.000-g-Harz-Maßstab aufgebaut und nach HF-Abspaltung in einer Rohpeptid-Reinheit von 85 bis 90 % erhalten werden. Der säurelabile Trp-Rest unterliegt bei Wahrung der 3 + 7-Strategie nicht der kritischen HF-Abspaltungsprozedur.

Im Gegensatz zu anderen Herstellungverfahren, in denen die 3 + 7-Kondensation mittels DCC oder DCC-Additiva durchgeführt wird, wird im erfindungsgemäßen Verfahren die Kupplung mit Chymotrypsin durchgeführt, wodurch eine Racemisierung des $Trp^3$-Restes ausgeschlossen wird. Dagegen wird bei den mit DCC/Additiva durchgeführten Kondensationen eine $Trp^3$-Racemisierung von 5 bis 15 % beobachtet. Die Racemisierung läßt sich auf <1 % senken, wenn die Kupplung über das Tripeptid-Azid durchgeführt wird. Die Ausbeuten bei diesem Verfahren liegen aber bei 20 bis 50 % (siehe z.B. DE 23 07 010), während bei der erfindungsgemäßen enzymatischen Verknüpfung bei Anwendung eines Tripeptidüberschusses von 1,3 Äquivalenten eine quantitative Umsetzung des eingesetzten Heptapeptides erreicht wird. Die enzymatische Kupplung hat darüber hinaus den Vorteil, daß Acylierungen an den ungeschützten Peptidseitenketten nicht möglich sind und die Reinheit des Dekapeptid-Rohproduktes ausschließlich von der Reinheit der eingesetzten Fragmente II und III abhängt. Die Endreinigung der erhaltenen Rohprodukte ist somit in einfacher Weise möglich, zumal die Abtrennung des Fragmentes II und der bei der enzymatischen Synthese gebildeten Tripeptidsäure sowohl durch Ionenaustauschchromatographie als auch durch reversed-phase Chromatographie an hydrophoben Polymeren problemlos ist.

Die folgenden Beispiele sollen das erfindungsgemäße Verfahren zur Herstellung von GnRH-Peptiden näher erläutern:

Ausführungsbeispiele

Beispiel 1

Herstellung von Pyr-His-Trp-Ser-Tyr-DPhe-Leu-Arg-Pro-Gly-$NH_2$:
21 g Pyr-His-Trp-OEt (43,70 mMol) und 20 g H-Ser-Tyr-DPhe-Leu-Arg-Pro-Gly-$NH_2$ (23,87 mMol) werden unter Rühren bei Raumtemperatur in 66 ml DMF und 70,5 ml Wasser suspendiert und der pH mit 2N KOH auf 8,0 gestellt. Dazu werden 450 $\mu$l einer Lösung von 50 mg $^\alpha$-Chymotrypsin (SERVA, 45 U/mg) in 1 ml $1 \cdot 10^{-3}$ N HCl gegeben. Die Enzymgabe wird nach 2 h wiederholt. Unter pH-stat.-Bedingungen (pH 8,0 mit 2 N KOH) wird nach 6,5 h ein nahezu quantitativer Umsatz des Heptapeptidamids erreicht (HPLC-Kontrolle) und die Enzamreaktion durch Zugabe von 10 ml Essigsäure abgebrochen. Mit 3 x 50 ml Methanol wird im Vakuum eingeengt und der ölige Rückstand in 3 l Aceton eingerührt.
Das ausgefällte Syntheseprodukt wird abgesaugt; mit Aceton und Ether gewaschen und getrocknet.
Ausbeute: 24,5 g
Überschüssig eingesetztes Pyr-His-Trp-OEt kann aus dem Acetonüberstand der Produktfällung durch Einengen und nach Auswaschen von gebildeten Pyr-His-Trp-OH mit Hydrogencarbonatlösung isoliert und wiederverwendet werden.

Beispiel 2

Wie Beispiel 1, nur anstelle von $^\alpha$-Chymotrypsin (SERVA, 45 U/mg) wird $^\alpha$-Chymotrypsin vom VEB Berlin-Chemie (275 U/mg, N-Acetyl-L-Tyrosinethylester) eingesetzt.
Ausbeute: 24,4 g

Beispiel 3

Wie Beispiel 1, nur anstelle von 23,87 mMol werden 0,75 mMol Heptapeptidamid eingesetzt und anstelle von synthetisierten Pyr-His-Trp-OEt wird rückgewonnenes Pyr-His-Trp-OEt eingesetzt.
Ausbeute 0,79 g

Beispiel 4

Wie Beispiel 1, nur anstelle von 450 µl (22,5 mg) gelöstem $^\alpha$-Chymotrypsin wird 200 mg immobiliertes $^\alpha$-Chymotrypsin (20 U/mg, N-Acetyl-L-Tyrosinethylester, pH 8,0, 25 °C) eingesetzt.
Ausbeute 24,2 g

**Patentansprüche**

1. Verfahren zur Herstellung von GnRH-Dekapeptiden der Formel I

   Pyr-His-Trp-Ser-Tyr-Xaa-Leu-Arg-Pro-Gly-
   NH$_2$      I

   wobei Xaa = DPhe oder eine andere D-Aminosäure bedeutet, dadurch gekennzeichnet, daß der Aufbau der GnRH-Dekapeptide aus den Fragmenten der Formel I und II

   Pyr-His-Trp-OR      I,

   H-Ser-Tyr-Xaa-Leu-Arg-Pro-Gly-NH$_2$      II,

   worin R Alkyl oder Aryl bedeutet und Xaa die oben genannte Bedeutung hat, durch Chymotrypsin-katalysierte Kupplung erfolgt.

2. Verfahren nach Anspruch 1, dadurch gekennzeichnet, daß die Reaktion bei einer Temperatur zwischen -10 °C und 35 °C durchgeführt wird.

3. Verfahren nach Anspruch 1 und 2, dadurch gekennzeichnet, daß als organisches Lösungsmittel Dimethylformamid eingesetzt wird.

4. Verfahren nach Anspruch 1 bis 3, dadurch gekennzeichnet, daß die Fragmente in der Wasser/DMF-Mischung auf einen pH = 8,5 gebracht werden, die Reaktion durch Zugabe des Enzyms gestartet wird und in einem Zeitraum von 2 h weiteres Enzym zugefügt wird.

5. Verfahren nach Anspruch 1 bis 4, dadurch gekennzeichnet, daß an einem polymeren Träger gebundenes Chymotrypsin eingesetzt wird.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl.⁵) |
|---|---|---|---|
| P,X | CHEMICAL ABSTRACTS, vol. 114, No. 13, April 1, 1991, Columbus, Ohio, USA V. SCHELLENBERGER et al. "Chymotrypsin-catalyzed fragment coupling synthesis of D-Phe(6)-GnRH" page 869, left column, abstract-No. 123 063c & Tetrahedron. Lett. 1990, 31(50) 7305-6 -- | 1-5 | C 12 P 21/02 A 61 K 37/38 A 61 K 37/02 C 07 K 7/06 |
| A | EP - A2 - 0 220 654 (RICHTER GEDEON VEGYESZETI GYAR) * Examples * -- | 1 | |
| A | GB - A - 2 185 025 (INNOFINANCE) * Examples; claims * -- | 1-5 | |
| A | EP - A2 - 0 122 712 (THE SALK INSTITUTE) * Abstract; example * ---- | 1 | |

RECHERCHIERTE SACHGEBIETE (Int. Cl.⁵)

A 61 K
C 07 K
C 12 P

Der vorliegende Recherchenbericht wurde für alle Patentansprüche erstellt.

| Recherchenort | Abschlußdatum der Recherche | Prüfer |
|---|---|---|
| WIEN | 01-10-1991 | AUGUSTIN |